# EUROPÄISCHE PATENTSCHRIFT

(11) **EP 1 857 078 B1**
(45) Veröffentlichungstag und Bekanntmachung des Hinweises auf die Patenterteilung: **09.03.2016**
(21) Anmeldenummer: 07006274.0
(22) Anmeldetag: 27.03.2007
(51) Int. Cl.: A61F 2/18, A61F 5/08

(54) **Implantat zur Spreizung der Nasenflügel**
Implant for splaying alar wings of the nose
Implant destiné à l'écartement de l'aile du nez

(30) Priorität: 17.05.2006 DE 102006023058
(43) Veröffentlichungstag der Anmeldung: 21.11.2007
(73) Patentinhaber: Heinz Kurz GmbH Medizintechnik, 72144 Dusslingen (DE)
(72) Erfinder: àWengen, Daniel, Dr., 4102 Binningen (CH); Steinhardt, Uwe, 72145 Hirrlingen (DE)
(74) Vertreter: Kohler Schmid Möbus Patentanwälte

(56) Entgegenhaltungen:
- EP-A- 1 475 056
- US-A- 5 133 754
- US-A- 5 716 405
- US-A1- 2002 173 848
- US-B1- 6 322 590
- US-B1- 6 454 803

## Beschreibung

Die Erfindung betrifft ein dachförmiges Implantat zur Spreizung der Nasenflügel, das am Dreiecksknorpel der menschlichen Nase befestigbar ist und aus einem zunächst ebenen, in eine Dachform gebogenen Streifen gefertigt ist.

Ein solches Implantat zur Spreizung der Nasenflügel ist beispielsweise durch die EP 1 475 056 A1 bekannt geworden.

Die Einpflanzung eines Implantats, d. h. eines in der Regel körperfremden Gewebsstücks oder Stoffes, in den menschlichen Körper ist in der Medizintechnik ein lange bekanntes Verfahren, das in vielen Variationen zur Behebung funktioneller Störungen verschiedener Körperteile und/oder psychischer Beeinträchtigungen vorgenommen wird.

Eine Spreizung der Nasenflügel kann beispielsweise bei einer Verengung der Nasenklappe oder bei einem Kollaps der Weichteile der Nasenflügel indiziert sein.

Eine einfache und bekannte Methode zur Spreizung der Nasenflügel besteht darin, ein Nasenstützpflaster zu verwenden, das insbesondere auch Hochleistungssportler zur Verbesserung der Nasenatmung einsetzen. Dieses Vorgehen ist jedoch zur dauerhaften Anwendung ungeeignet. Bei regelmäßiger Verwendung der Nasenpflaster können durch den Klebstoff Hautprobleme entstehen. Außerdem beeinträchtigt ein Nasenpflaster das äußere Erscheinungsbild eines Menschen.

Zur dauerhaften Spreizung der Nasenflügel sind daher auch operative Methoden bekannt, bei denen zur Stabilisierung der seitlichen Nasenweichteile Knorpel eingesetzt wird. Die Ergebnisse sind jedoch optisch und funktional nicht immer befriedigend. Außerdem bringt dieses Vorgehen noch andere Nachteile mit sich. Da vorzugsweise körpereigener Knorpel zur Stabilisierung verwendet wird, muss die Entnahme des Knorpels, vorzugsweise aus dem Ohr oder aus der Nasenscheidewand des Patienten, diesem Schritt vorangehen, was einen zeitlichen Aufwand und ein zusätzliches Risiko für den Patienten bedeutet. Oft zeigt sich, dass das eingesetzte Teil zu wenig Eigenspannung besitzt, um den kaudalen Luftraum der Nase in einer befriedigenden Weise offen zu halten. Das Verfahren kann sich sogar als kontraproduktiv erweisen, weil die eingesetzten Teile die Weichteile nach innen drücken und zu einer weiteren Verengung der Nase führen können.

Auch metallische Implantate werden zur Spreizung der Nasenflügel eingesetzt. Dazu wird operativ eine Öffnung im Bereich des Flügelrandes der Nase eingebracht, durch welche das Implantat eingesetzt und am Dreiecksknorpel befestigt wird. Metallische Implantate sind wesentlich fester und elastischer als Knorpel und besitzen genügend Eigenspannung, um den nasalen Luftraum in einer dauerhaften und befriedigenden Weise offen zu halten.

Aus der US 6,322,590 B1 ist ein dachförmiges Implantat bekannt, das aus einem geraden ebenen Metallstreifen gefertigt ist. Dieser Metallstreifen weist an seinen beiden Enden Perforationen in Form von runden Löchern auf und wird aus der Ebene in eine räumlich V-förmige Dachform, allerdings mit "runder Spitze" des räumlichen V gebogen.

Andere Implantierungsanordnungen in der Nase ermöglichen Implantate nach der EP 1 475 056 A1, bei welchen der ebene Streifen ebenfalls aus der Ebene in eine räumlich V-förmige Dachform gebogen wird, jedoch in der Ebene bereits eine winkelförmige Kontur, insbesondere eine V-förmige oder trapezförmige Kontur, aufweist.

Nun weist der Dreiecksknorpel der menschlichen Nase bekanntlich einen relativ flachen oder allenfalls sehr leicht gekrümmten Plateaubereich auf. Nachteilig bei allen bekannten Implantaten ist, dass sie um den Nasenrücken herum jeweils so stark gekrümmt sind, dass im implantierten Zustand im Bereich dieses Plateaus des Dreiecksknorpels ein Hohlraum zwischen Dreiecksknorpel und dem Implantat entsteht, der zu einem ungünstigen Abheilverhalten nach der Implantation und möglicherweise zu unkontrolliert wuchernden Gewebeansammlungen führen kann. Des Weiteren sind die bekannten Implantate geometrisch so gestaltet, dass sie nicht optimal an den Flanken des Dreiecksknorpels im Bereich der Nasenflügel anliegen.

Der Erfindung liegt daher die Aufgabe zugrunde, mit unaufwändigen technischen Mitteln ein Implantat der eingangs genannten Art dahingehend zu modifizieren, dass diese Nachteile vermieden werden und das Implantat nach der Operation möglichst eng am Dreiecksknorpel anliegt.

Diese Aufgabe wird auf überraschend einfache und kostengünstig zu realisierende Weise dadurch gelöst, dass ein Rückenabschnitt des Implantats oberhalb des Nasenrückens flach oder nur sehr leicht gegen den Plateaubereich des Dreiecksknorpels mit einem Spreizungswinkel ω>160° abgewinkelt oder tonnenförmig mit einem Krümmungsradius r>4cm, vorzugsweise r>10cm, um den Plateaubereich des Dreiecksknorpels herum gekrümmt ist, und dass zwei Seitenabschnitte des Implantats beiderseits der Nasenflügel im Wesentlichen parallel zum jeweiligen Nasenflügel unter einem Winkel ϕ von jeweils mehr als 50° gegen den flachen Rückenabschnitt nach unten abgekantet verlaufen.

Damit wird ohne größeren Fertigungsaufwand eine besonders gute geometrische Anpassung des Implantats an die (normale) Ausgestaltung des menschlichen Dreiecksknorpels erreicht. Insbesondere entstehen nach der Operation keine Hohlräume zwischen dem Implantat und dem Dreiecksknorpel, weder im Bereich des Plateaus des Dreiecksknorpels noch an dessen Flanken. Vielmehr liegt das Implantat über seine gesamte dem Dreiecksknorpel zugewandte Oberfläche eng an diesem an, was auch einen besonders guten mechanischen Halt des Implantats am Dreiecksknorpel bewirkt.

In einer bevorzugten Ausführungsform der Erfindung sind die beiden Seitenabschnitte des ebenen Streifens zu ihren freien Enden hin verbreitert. Durch diese Maßnahme können die Nasenflügel großflächig gespreizt bzw. stabilisiert werden.

Als Material für chirurgische und orthopädische Implantate sind Metalle und deren Legierungen prädestiniert, weil sie neben einer sehr guten Biokompatibilität hohe Dauerfestigkeit und Elastizität aufweisen. Trotz einer relativ geringen Dichte besitzen Implantate aus solchen Materialien wie Titan oder Titanverbindungen ausgezeichnete mechanische Eigenschaften mit einer langen Lebensdauer. Ebenfalls eine gute Eignung für die genannten Zwecke besitzt Edelstahl. Das erfindungsgemäße Implantat kann zwar prinzipiell auch aus einem geeigneten Kunststoff bestehen, wird aber vorzugsweise aus Metall, insbesondere aus Titan, einer Titanlegierung oder Edelstahl gefertigt sein.

Für die Form des Implantats gibt es mehrere Möglichkeiten. In einer einfachen Ausführung kann der ebene Streifen, aus dem das Implantat aufgebaut ist, in der Ebene V-förmig gebogen sein, wobei zweckmäßigerweise die Spitze des V abgerundet ist. Der ebene Streifen kann auch trapezförmig gestaltet sein und/oder kompliziertere Strukturen mit Verzweigungen aufweisen, die nach dem Aufbiegen des Implantats in seine räumliche Endform zu einer Stabilisierung beispielsweise des Nasenrückens dienen können.

In einer vorteilhaften Ausgestaltung können der ebene Streifen bzw. das fertige Implantat Perforationen aufweisen. Damit wird einerseits das Gewicht des Implantats verringert, andererseits der Anteil von körperfremdem Material, das durch das Implantat in den menschlichen Körper eingebracht wird, so weit wie möglich reduziert. Außerdem fördern die Perforationen das Verwachsen des Implantats mit dem Gewebe. Die Perforationen sind vorzugsweise sowohl an den Seitenabschnitten des Implantats als auch an dem dazwischen liegenden Rückenabschnitt vorgesehen und zum Beispiel als kreisrunde Löcher oder als Langlöcher ausgebildet. Weiter dienen die Perforationen einer sicheren Fixierung am Dreiecksknorpel mittels einer Naht.

Zusätzlich zur guten Biokompatibilität des verwendeten Materials selbst können der ebene Streifen bzw. das Implantat auch einen besonderen, körperverträglichen Überzug aufweisen.

Zur Erzielung einer fein abgestimmten Form des ebenen Streifens bzw. des Implantats können diese zweckmäßigerweise mittels Lasertechnik hergestellt sein.

Ganz besonders bevorzugte Ausführungsformen der Erfindung zeichnen sich dadurch aus, dass die freien Enden der beiden Seitenabschnitte des Implantats um einen noch größeren Winkel gegen den Rückenabschnitt nach unten abgekantet sind als die übrigen Teile der Seitenabschnitte. Auf diese Weise wird eine besonders Anlage an den Dreiecksknorpel erreicht.

Bei vorteilhaften Weiterbildungen dieser Ausführungsformen sind die Abkantungswinkel der freien Enden der beiden Seitenabschnitte so gestaltet, dass sich die Seitenabschnitte im implantierten Zustand in einem engen räumlichen Kontakt, insbesondere in einer beiderseits unter Spannung stehenden, vorzugsweise symmetrischen Verklemmung mit dem Dreiecksknorpel befinden, was zu einem besonders guten Sitz des Implantats beiträgt.

Bei weiteren vorteilhaften Ausführungsformen der Erfindung sind der ebene Streifen oder das Implantat im Spritzguss nach dem Micro Injection Moulding (=MIM) Verfahren gefertigt, welches beispielsweise aus der WO 00/06327 A2 an sich bekannt ist. Damit kann eine äußerst preisgünstige Herstellung auch sehr großer Stückzahlen bei gleich bleibender Maßgenauigkeit erreicht werden, während die herkömmlichen Implantate in der Regel wie etwa Schmuckwaren handangefertigt werden und daher einerseits relativ teuer in der Herstellung sind, andererseits in der Maßgenauigkeit individuell variieren können.

Besonders bevorzugt schließlich sind auch Ausführungsformen der Erfindung, bei denen der ebene Streifen oder das Implantat aus einem Werkstoff mit superelastischen Eigenschaften, vorzugsweise aus Nitinol, gefertigt sind, so dass beispielsweise durch geeignete thermische Behandlung dem Implantat optimale Federeigenschaften relativ zum Dreiecksknorpel eingebracht werden können.

Weitere Merkmale und Vorteile der Erfindung ergeben sich aus der nachfolgenden detaillierten Beschreibung von Ausführungsbeispielen der Erfindung anhand der Figuren der Zeichnung, die erfindungswesentliche Einzelheiten zeigt, sowie aus den Ansprüchen. Die einzelnen Merkmale können je einzeln für sich oder zu mehreren in beliebigen Kombinationen bei Varianten der Erfindung verwirklicht sein.

In der schematischen Zeichnung sind Ausführungsbeispiele der Erfindung dargestellt, welche in der nachfolgenden Beschreibung näher erläutert werden.

Es zeigen:
- Fig. 1: eine perspektivische Ansicht einer ersten Ausführungsform eines erfindungsgemäßen Implantats mit flachem Rückenabschnitt;
- Fig. 2a: eine schematische Frontalansicht auf das Implantat aus Fig. 1 in einer Richtung parallel zur Oberkante des Nasenrückens gesehen;
- Fig. 2b: eine schematische Frontalansicht auf eine zweite Ausführungsform eines erfindungsgemäßen Implantats mit leicht gegen den Plateaubereich des Dreiecksknorpels abgewinkeltem Rückenabschnitt; und
- Fig. 3: eine schematische Ansicht eines ebenen Zuschnitt für ein erfindungsgemäßes Implantat.

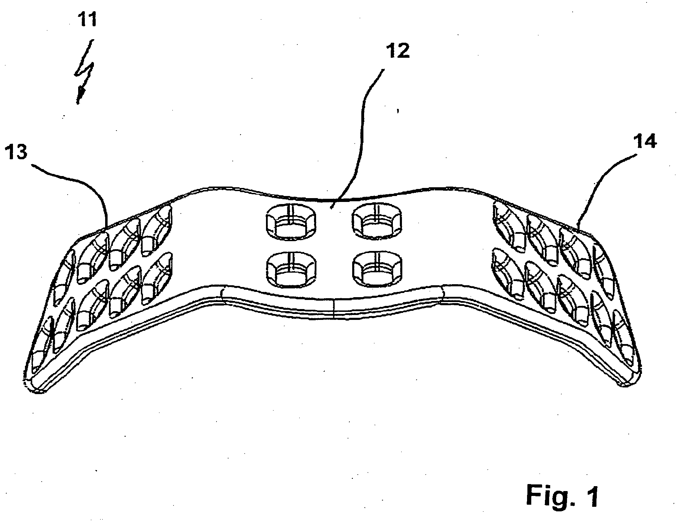

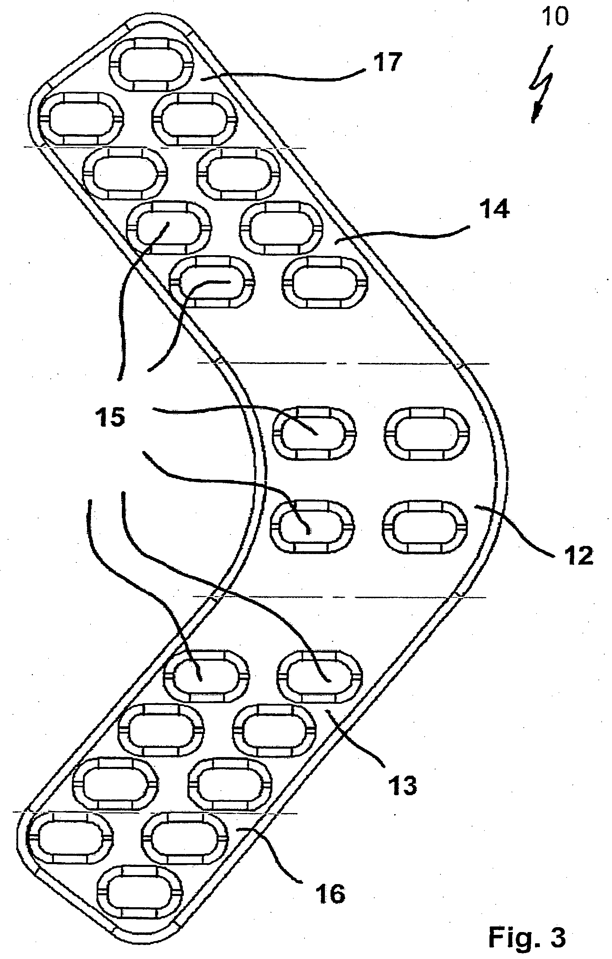

Fig. 1 zeigt in einer perspektivischen Ansicht eine erste mögliche Ausführungsform eines erfindungsgemäßen Implantats **11.** Dieses ist aus einem ebenen Streifen **10,** wie er in Fig. 3 schematisch dargestellt ist, in eine dachartige Raumform gebogen.

Bereits in der Ebene ist der Streifen 10 V-artig gebogenen und mit einer abgerundeten Spitze versehen. Er weist eine Reihe von regelmäßigen Perforationen **15** auf, welche helfen, einerseits das Gewicht des Implantats 11 zu verringern und andererseits den Anteil des körperfremden Materials im Körper eines Patienten so weit wie möglich zu reduzieren. Außerdem fördern die Perforationen 15 das Verwachsen des Implantats 11 mit dem Gewebe. Das Implantat 11 wird operativ durch eine so genannte offene Rhinoplastik unter den Flügelknorpel in die Nase eingebracht und auf dem Dreiecksknorpel mittels einer Naht befestigt. Hierbei werden mehrere Einzelnähte durch die Perforationen und den Dreiecksknorpel angelegt und fixiert.

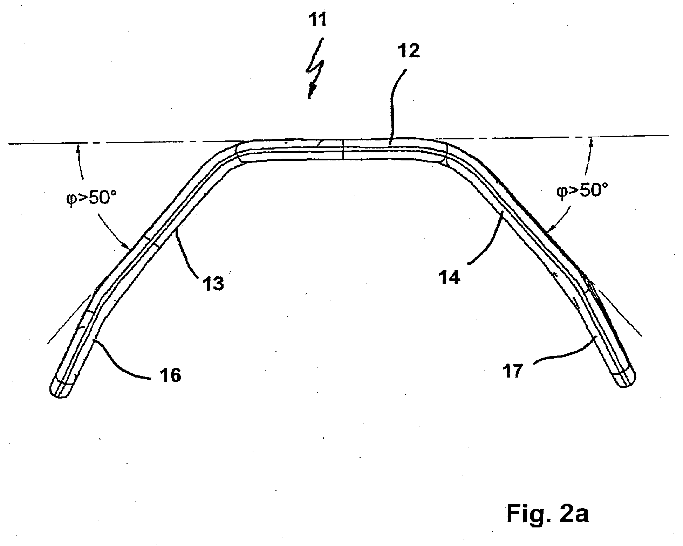

In den Fig. 1 und 2a ist gut zu erkennen, dass die dort dargestellte Ausführungsform des erfindungsgemäßen Implantats 11 einen flachen Rückenabschnitt **12** aufweist, der im implantierten Zustand oberhalb des Nasenrückens angeordnet ist. Beiderseits der Nasenflügel verlaufen zwei Seitenabschnitte **13, 14** des Implantats 11 im Wesentlichen parallel zum jeweiligen Nasenflügel unter einem Winkel ϕ von jeweils mehr als 50° gegen den flachen Rückenabschnitt 12 nach unten abgekantet, wie insbesondere in Fig. 2a angedeutet ist. Die freien Enden **16, 17** der beiden Seitenabschnitte 13, 14 des Implantats 11 verlaufen um einen noch größeren Winkel gegen den Rückenabschnitt 12 nach unten abgekantet als die übrigen Teile der Seitenabschnitte 13, 14, wobei die Abkantungswinkel der freien Enden 16, 17 so gestaltet sind, dass sich die Seitenabschnitte 13, 14 im implantierten Zustand in einem engen räumlichen Kontakt, insbesondere in einer beiderseits unter Spannung stehenden, vorzugsweise symmetrischen Verklemmung mit dem Dreiecksknorpel befinden.

Der Rückenabschnitt des erfindungsgemäßen Implantats muss aber nicht 100%ig flach gestaltet sein. Er kann bei Ausführungsformen auch eine sehr leichte Abwinkelung oder eine ganz geringe Krümmung aufweisen, ohne dass dabei die Vorteile der Erfindung insgesamt verloren gehen. Im Gegenteil ist ja bei manchen Menschen der plateauförmige Bereich des Dreiecksknorpels ebenfalls nicht völlig flach, sondern ganz leicht gewölbt, so dass mit solchen Ausführungsformen eine besonders gute geometrische Anpassung des Implantats an die individuellen Gegebenheiten des Patienten vorgenommen werden kann.

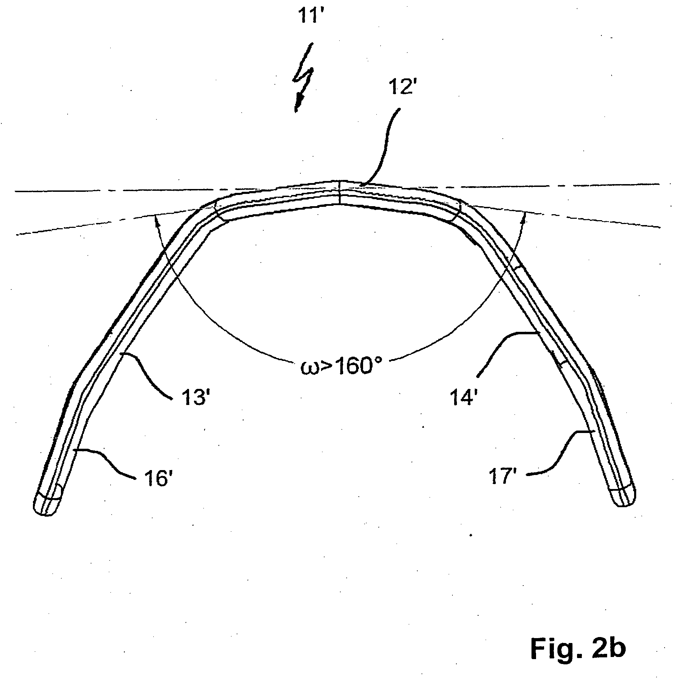

In Fig. 2b ist daher eine Ausführungsform dargestellt, bei der das Implantat **11'** einen nur sehr leicht gegen den Plateaubereich des Dreiecksknorpels mit einem Spreizungswinkel ω>160° abgewinkelten Rückenabschnitt **12'** aufweist. Auch hier verlaufen die beiden Seitenabschnitte **13', 14'** des Implantats 11' im Wesentlichen parallel zum jeweiligen Nasenflügel unter einem Winkel ϕ von jeweils mehr als 50° gegen den flachen Rückenabschnitt 12' nach unten abgekantet und laufen wiederum in freie Enden **16', 17'** aus, die um einen noch größeren Winkel gegen den Rückenabschnitt 12' nach unten abgekantet sind als die übrigen Teile der Seitenabschnitte 13', 14'.

Anstelle der in Fig. 2b gezeigten leichten Abwinkelung kann der Rückenabschnitt des erfindungsgemäßen Implantats bei weiteren, in der Zeichnung nicht eigens dargestellten Ausführungsformen auch eine ganz leichte tonnenförmige Krümmung mit einem relativ großen Krümmungsradius r>4cm, vorzugsweise sogar r>10cm, um den Plateaubereich des Dreiecksknorpels herum aufweisen.

## Patentansprüche

1. Dachförmiges Implantat zur Spreizung der Nasenflügel, das am Dreiecksknorpel der menschlichen Nase befestigbar ist und aus einem zunächst ebenen, in eine Dachform gebogenen Streifen (10) aus Metall gefertigt ist, wobei ein zentraler Rückenabschnitt (12; 12') des Implantats (11; 11') flach oder nur sehr leicht von einer Horizontalebene nach unten abgewinkelt oder tonnenförmig von der Horizontalebene nach unten gekrümmt ist, und wobei zwei Seitenabschnitte (13, 14; 13',14') des Implantats (11; 11') beiderseits des zentralen Rückenabschnitts (12; 12') symmetrisch zum zentralen Rückenabschnitt (12; 12')gegen den zentralen Rückenabschnitt (12; 12') nach unten abgekantet verlaufen,
**dadurch gekennzeichnet, dass** der zentrale Rückenabschnitt (12; 12') des Implantats (11; 11') von einer Horizontalebene nach unten mit einem Spreizungswinkel ω>160° abgewinkelt oder tonnenförmig mit einem Krümmungsradius r>4cm, vorzugsweise r>10cm, von der Horizontalebene nach unten gekrümmt ist, dass zwei Seitenabschnitte (13, 14; 13',14') des Implantats (11; 11') beiderseits des zentralen Rückenabschnitts (12; 12') symmetrisch zum zentralen Rückenabschnitt (12; 12') unter einem Winkel ϕ von jeweils mehr als 50° gegen den zentralen Rückenabschnitt (12; 12') nach unten abgekantet verlaufen, und dass die freien Enden (16, 17; 16', 17') der beiden Seitenabschnitte (13, 14; 13', 14') des Implantats (11; 11') um einen noch größeren Winkel gegen den Rückenabschnitt (12; 12') nach unten abgekantet verlaufen als die übrigen Teile der Seitenabschnitte (13, 14; 13', 14').

2. Implantat nach Anspruch 1, **dadurch gekennzeichnet, dass** die beiden Seitenabschnitte (13, 14; 13', 14') des ebenen Streifens (10) zu ihren freien Enden hin verbreitert sind.

3. Implantat nach einem der vorhergehenden Ansprüche, **dadurch gekennzeichnet, dass** der ebene Streifen (10) oder das Implantat (11; 11') aus Titan, aus einer Titanlegierung oder aus Edelstahl gefertigt ist.

4. Implantat nach einem der vorhergehenden Ansprüche, **dadurch gekennzeichnet, dass** der ebene Streifen (10) eine winkelförmige Kontur, insbesondere eine V-förmige oder trapezförmige Kontur mit oder ohne Verzweigungen aufweist.

5. Implantat nach einem der vorhergehenden Ansprüche, **dadurch gekennzeichnet, dass** der ebene Streifen (10) oder das Implantat (11; 11') Perforationen (15) aufweist.

6. Implantat nach Anspruch 5, **dadurch gekennzeichnet, dass** die Perforationen (15) sowohl an den Seitenabschnitten (13, 14; 13', 14') als auch an dem dazwischen liegenden Rückenabschnitt (12; 12') des Implantats (11; 11') vorgesehen sind.

7. Implantat nach Anspruch 5 oder 6, **dadurch gekennzeichnet, dass** die Perforationen (15) als kreisrunde Löcher oder als Langlöcher ausgebildet sind.

8. Implantat nach einem der vorhergehenden Ansprüche, **dadurch gekennzeichnet, dass** der ebene Streifen (10) oder das Implantat (11; 11') einen körperverträglichen Überzug aufweist.

9. Implantat nach einem der vorhergehenden Ansprüche, **dadurch gekennzeichnet, dass** der ebene Streifen (10) oder das Implantat (11; 11') mittels Lasertechnik hergestellt ist.

10. Implantat nach einem der vorhergehenden Ansprüche, **dadurch gekennzeichnet, dass** die Abkantungswinkel der freien Enden (16,17; 16',17') der beiden Seitenabschnitte (13, 14; 13', 14') geometrisch so gestaltet sind, dass sich die Seitenabschnitte (13, 14; 13', 14') im implantierten Zustand in einem engen räumlichen Kontakt, insbesondere in einer beiderseits unter Spannung stehenden, vorzugsweise symmetrischen Verklemmung mit dem Dreiecksknorpel befinden können.

11. Implantat nach einem der vorhergehenden Ansprüche, **dadurch gekennzeichnet, dass** der ebene Streifen (10) oder das Implantat (11; 11') im Spritzguss nach dem Micro Injection Moulding (=MIM) Verfahren gefertigt ist.

12. Implantat nach einem der vorhergehenden Ansprüche, **dadurch gekennzeichnet, dass** der ebene Streifen (10) oder das Implantat (11; 11') aus einem Werkstoff mit Memory-Effekt, vorzugsweise aus Nitinol, gefertigt ist.

## Claims

1. A roof-shaped implant for splaying the nasal alae (wings), which implant can be attached to the triangular cartilage of the human nose and is made from an initially flat metal strip (10) bent into a roof shape, a central rear portion (12; 12') of the implant (11; 11') being flat, or angled only very slightly downwards from a horizontal plane, or curved downwards in the shape of a dome from the horizontal plane, and two side portions (13, 14; 13', 14') of the implant (11; 11'), on both sides of the central rear portion (12; 12'), extending downwards symmetrically relative to the central rear portion (12; 12') at an angle relative to the central rear portion (12; 12'),
**characterized in that** the central rear portion (12; 12') of the implant (11; 11') is angled downwards from the horizontal plane with an angle of spread of ω>160°, or curved downwards from the horizontal plane in the shape of a dome with a radius of curvature of r>4cm, and preferably r>10cm, **in that,** on both sides of the central rear portion (12; 12'), two side portions (13, 14; 13', 14') of the implant (11; 11') each extend downwards symmetrically relative to the central rear portion (12; 12') at an angle φ of more than 50° relative to the central rear portion (12; 12'), **and in that** the free ends (16, 17; 16', 17') of the two side portions (13, 14; 13', 14') of the implant (11; 11') are angled downwards at a still greater angle relative to the rear portion (12; 12') than the other parts of the side portions (13, 14; 13', 14').

2. An implant according to Claim 1, **characterized in that** the two side portions (13, 14; 13', 14') of the flat strip (10) are widened towards their free ends.

3. An implant according to one of the preceding claims, **characterized in that** the flat strip (10) or implant (11; 11') is made of titanium, a titanium alloy or of stainless steel.

4. An implant according to one of the preceding claims, **characterized in that** the flat strip (10) has an angular contour, in particular a V-shaped or trapezoidal contour with or without branching portions.

5. An implant according to one of the preceding claims, **characterized in that** the flat strip (10) or implant (11; 11') has perforations (15).

6. An implant according to Claim 5, **characterized in that** the perforations (15) are provided in the side portions (13, 14; 13', 14') as well as in the intermediate rear portion (12; 12') of the implant (11, 11').

7. An implant according to Claim 5 or 6, **characterized in that** the perforations (15) take the form of circular holes or slots.

8. An implant according to one of the preceding claims, **characterized in that** the flat strip (10) or implant (11, 11') has a biocompatible coating.

9. An implant according to one of the preceding claims, **characterized in that** the flat strip (10) or implant (11; 11') is produced using laser technology.

10. An implant according to one of the preceding claims, **characterized in that** the bending angles of the free ends (16, 17; 16', 17') of the two side portions (13, 14; 13', 14') are geometrically configured in such a way that, in the implanted state, the side portions (13, 14; 13', 14') can establish close spatial contact with the triangular cartilage, in a preferably symmetrical clamping arrangement which is under tension on both sides.

11. An implant according to one of the preceding claims, **characterized in that** the flat strip (10) or implant (11; 11') is produced using injection moulding according to the micro injection moulding (=MIM) process.

12. An implant according to one of the preceding claims, **characterized in that** the flat strip (10) or implant (11,11') is made of a material with memory effect, preferably nitinol.

## Revendications

1. Implant en forme de toit destiné à l'écartement des ailes du nez, qui peut être fixé sur le cartilage triangulaire du nez humain et qui est fabriqué à partir d'une bande (10) en métal tout d'abord plane arquée en forme de toit, dans lequel un tronçon dorsal (12 ; 12') central de l'implant (11 ; 11') est plat ou uniquement très légèrement plié vers le bas à partir d'un plan horizontal ou courbé vers le bas en forme de tonneau à partir du plan horizontal, et dans lequel deux tronçons latéraux (13, 14 ; 13', 14') de l'implant (11 ; 11') s'étendent de façon repliée vers le bas des deux côtés du tronçon dorsal (12 ; 12') central symétriquement au tronçon dorsal (12 ; 12') central contre le tronçon dorsal (12 ; 12') central,
**caractérisé en ce que** le tronçon dorsal (12 ; 12') central de l'implant (11 ; 11') est plié vers le bas à partir d'un plan horizontal avec un angle d'écartement ω > 160° ou courbé vers le bas à partir du plan horizontal en forme de tonneau avec un rayon de courbure r > 4 cm, de préférence r > 10 cm, **en ce que** deux tronçons latéraux (13, 14 ; 13', 14') de l'implant (11 ; 11') s'étendent de façon repliée vers le bas des deux côtés du tronçon dorsal (12 ; 12') central symétriquement au tronçon dorsal (12 ; 12') central en formant un angle ϕ de respectivement plus de 50° contre le tronçon dorsal (12 ; 12') central, et **en ce que** les extrémités libres (16, 17 ; 16', 17') des deux tronçons latéraux (13, 14; 13', 14') de l'implant (11 ; 11') s'étendent de façon repliée vers le bas contre le tronçon dorsal (12 ; 12') selon un angle encore plus grand que les autres parties des tronçons latéraux (13, 14 ; 13', 14').

2. Implant selon la revendication 1, **caractérisé en ce que** les deux tronçons latéraux (13, 14 ; 13', 14') de la bande (10) plane sont élargis en direction de leurs extrémités libres.

3. Implant selon l'une des revendications précédentes, **caractérisé en ce que** la bande (10) plane ou l'implant (11 ; 11') est fabriqué(e) en titane, en alliage de titane ou en acier fin.

4. Implant selon l'une des revendications précédentes, **caractérisé en ce que** la bande (10) plane présente un contour de forme angulaire, en particulier un contour en forme de V ou de forme trapézoïdale avec ou sans ramifications.

5. Implant selon l'une des revendications précédentes, **caractérisé en ce que** la bande (10) plane ou l'implant (11 ; 11') présente des perforations (15).

6. Implant selon la revendication 5, **caractérisé en ce que** les perforations (15) sont prévues aussi bien sur les tronçons latéraux (13, 14 ; 13', 14') que sur le tronçon dorsal (12; 12') intercalé de l'implant (11 ; 11').

7. Implant selon la revendication 5 ou 6, **caractérisé en ce que** les perforations (15) sont réalisées en tant que trous circulaires ou en tant que trous oblongs.

8. Implant selon l'une des revendications précédentes, **caractérisé en ce que** la bande (10) plane ou l'implant (11 ; 11') présente un revêtement pouvant être toléré par le corps.

9. Implant selon l'une des revendications précédentes, **caractérisé en ce que** la bande (10) plane ou l'implant (11 ; 11') est fabriqué(e) par technique laser.

10. Implant selon l'une des revendications précédentes, **caractérisé en ce que** les angles de repli des extrémités libres (16, 17 ; 16', 17') des deux tronçons latéraux (13, 14 ; 13', 14') sont géométriquement constitués de telle sorte que, dans l'état implanté, les tronçons latéraux (13, 14 ; 13', 14') peuvent se trouver dans un état de contact spatial étroit avec le cartilage triangulaire, en particulier dans un état de coincement, de préférence symétrique, qui est sous tension des deux côtés.

11. Implant selon l'une des revendications précédentes, **caractérisé en ce que** la bande (10) plane ou l'implant (11 ; 11') est fabriqué(e) par moulage par injection selon le procédé micro injection moulding (MIM).

12. Implant selon l'une des revendications précédentes, **caractérisé en ce que** la bande (10) plane ou l'implant (11 ; 11') est fabriqué(e) dans un matériau à effet mémoire, de préférence en Nitinol.
